# EUROPÄISCHE PATENTSCHRIFT

(11) **EP 3 261 566 B1**
(45) Veröffentlichungstag und Bekanntmachung des Hinweises auf die Patenterteilung: **17.07.2019**
(21) Anmeldenummer: 15706811.5
(22) Anmeldetag: 26.02.2015
(51) Int. Cl.: A61B 17/80, A61B 17/86

(54) **KNOCHENPLATTE UND OPERATIONSSET ZUM FIXIEREN VON KNOCHENFRAGMENTEN**
BONE PLATE AND SURGICAL KIT FOR FIXING BONE FRAGMENTS
PLAQUE D'OSTÉOSYNTHÈSE ET KIT CHIRURGICAL POUR LA FIXATION DE FRAGMENTS OSSEUX

(43) Veröffentlichungstag der Anmeldung: 03.01.2018
(73) Patentinhaber: Medartis Holding AG, 4057 Basel (CH)
(72) Erfinder: THIEL, Dirk, 79219 Staufen (DE); ZUBERER, Alexander, 79400 Kandern (DE)
(74) Vertreter: Hepp Wenger Ryffel AG
(86) Internationale Anmeldenummer: PCT/EP2015/054055
(87) Internationale Veröffentlichungsnummer: WO 2016/134775

(56) Entgegenhaltungen:
- EP-A2- 1 093 762
- WO-A1-2004/086990
- US-A1- 2009 234 359
- US-A1- 2009 264 934

## Beschreibung

Die vorliegende Erfindung betrifft Knochenplatten und Operationssets zum Fixieren einer Knochenplatte. Für die Heilung von Knochenfrakturen mithilfe von Knochenplatten sind zwei Funktionen von besonderer Bedeutung, nämlich die Kompression und die Verblockung.

Während der Kompression werden zwei Knochenfragmente entlang eines Kompressionsweges aufeinander zu bewegt, so dass ein zwischen diesen Knochenfragmenten gebildeter Frakturspalt geschlossen wird. Eine solche Kompression an sich kann mit aus dem Stand der Technik bekannten besonders ausgestalteten Kompressionslöchern erreicht werden, welche derart ausgebildet sind, dass eine in den Knochen hinein gerichtete axiale Bewegung einer Knochenschraube in eine Lateralbewegung der Knochenschraube umgesetzt wird. Zu diesem Zweck enthalten die bekannten Knochenplatten Kompressionslöcher mit einer Kompressionskontur, die z.B. eine gegenüber der Plattenebene geneigte Gleitfläche aufweisen.

Die bekannten Knochenplatten werden zunächst mittels einer ersten Knochenschraube an einem ersten der beiden Knochenfragmente befestigt. Anschliessend wird eine zweite Knochenschraube durch das Kompressionsloch hindurch in einen zweiten der beiden Knochenfragmente eingesetzt. Beim Eindrehen der zweiten Knochenschraube gleitet die Unterseite des Schraubenkopfes an der Gleitfläche herunter, was eine Relativbewegung zwischen zweiter Knochenschraube und Knochenplatte und damit zwischen den beiden Knochenfragmenten bewirkt, also eine Kompression.

Unter einer ebenfalls an sich bekannten Verblockung wird die Fixierung eines Kopfes der Knochenschraube in der Knochenplatte verstanden. Hierdurch kann eine Knochenplatte winkelstabil an einem Knochen befestigt werden. Dabei ist es von grossem Vorteil, wenn die Knochenschraube unter einem variablen Winkel an der Knochenplatte befestigt werden kann, um der individuellen Anatomie des behandelten Patienten Rechnung tragen zu können.

Die US 7,354,441 B2 offenbart ein Kombinationsloch mit einem ersten, im Wesentlichen kreisförmigen Bereich und einem zweiten, länglichen Bereich. Der erste Bereich verfügt über ein Gewinde. Aufgrund der Kontur des Kombinationslochs erfolgt beim Eindrehen einer Knochenschraube eine Kompression der zu verbindenden Knochenfragmente.

Die WO 00/66012 A1 offenbart verblockbare Knochenplatten. In einem Ausführungsbeispiel wird eine Kompressions-Osteosynthese dargestellt. Durch Eindrehen von Knochenschrauben in ein Kompressionsloch werden die Knochenfragmente aufeinander zu bewegt, sodass sich ein dazwischen gebildeter Frakturspalt schliesst.

Die US 2008/0015592 A1 beschäftigt sich mit variabel verwendbaren Löchern: Wahlweise kann eine Kompressionsschraube eingesetzt werden, um eine Kompression zu bewirken, oder es kann eine Verblockungsschraube eingesetzt werden, um eine Verblockung zu erreichen. Zu diesem Zweck verfügen die Löcher über Kompressionsflächen und speziell geformte Kurvenbahnen. Zum Verblocken wird eine Knochenschraube verwendet, die über Nocken verfügt, welche entlang der Kurvenbahnen geführt werden. Für eine Kompression wird eine Knochenschraube ohne Nocken verwendet. Deren Kopf gleitet beim Eindrehen der Schraube die Kompressionsfläche herunter.

Die WO 2010/115403 A1 befasst sich mit der winkelstabilen Fixation und der Kompression einer Bruchstelle, wobei beide Funktionen gleichzeitig mit einer einzigen Knochenschraube erreicht werden sollen. Zu diesem Zweck verfügt die Knochenplatte über ein Fixations- und Kompressionsloch, welches aus zwei kreisförmigen Löchern gebildet ist, welche verschiedene Durchmesser aufweisen. Auf diese Weise entsteht eine sichelförmige Erweiterung. Im Loch ist ein Feingewinde vorgesehen. Beim Einschrauben dringt ein Aussengewinde der Knochenschraube seitlich in den offenen Teil des Innengewindes ein und erfasst dieses, sodass im eingeschraubten Zustand der Gewindekopf vollständig in dem Innengewinde aufgenommen ist.

Die Löcher der in EP 2 364 658 A1 offenbarten Knochenplatten enthalten einen ersten Bereich, einen zweiten Bereich und einen Übergangsbereich. Der Übergangsbereich ist dabei so ausgebildet, dass eine laterale Bewegung zwischen Platte und Schraube möglich ist. Zu diesem Zweck enthält der Übergangsbereich eine Abschrägung, welche zu einer Kompression führt.

Die WO 2011/160846 A1 offenbart Knochenplatten mit zwei verschieden grossen, sich schneidenden, stufigen Rundlöchern, in deren oberen Bereichen sich eine umlaufende Rippe erstreckt. Die erfindungsgemässen Knochenplatten sollen mit verschiedenartigen Knochenschrauben (mit Konussitzflächen oder Kugelsitzflächen) angewendet werden können. Eine Knochenschraube mit Gewinde am Schraubkopf kann sich an der Rippe des kleineren Rundlochs abstützen und einen Eingriff für die Rippe des grösseren Rundloches ermöglichen, wodurch eine gegenseitige Klemmung.

Auch die sehr ähnliche DE 10 2010 025 000 A1 offenbart Knochenplatten mit zwei verschieden grossen, sich schneidenden, stufigen Rundlöchern sowie einer radialen Rippe.

Die WO 2012/000627 A1 offenbart ebenfalls eine Knochenplatte mit zwei verschieden grossen, sich schneidenden, stufigen Rundlöchern, in deren oberen Bereichen sich eine Rippe erstreckt.

Auch die US 2012/0197307 A1 offenbart ein kombiniertes Schraubenloch zum Verblocken und Komprimieren.

Die WO 2004/086990 A1 offenbart Aufnahmeöffnungen für Knochenplatten, die eine Verblockung der Knochenschrauben unter verschiedenen Winkeln relativ zur Knochenplatte erlauben. Einige Ausführungsformen verfügen über ein Langloch mit Verblockungskontur, die für die Kompressionsosteosynthese eingesetzt werden kann.

Das bei all diesen Knochenplatten genutzte Konzept des Heruntergleitens der Unterseite des Schraubenkopfes an einer Gleitfläche des Kompressionslochs weist jedoch eine Reihe von Nachteilen auf. Denn wird der Winkel zwischen Gleitfläche und Plattenebene zu flach gewählt, so müssen grosse Kräfte in der Eindrehrichtung aufgebracht werden, um überhaupt eine Lateralbewegung der Knochenschraube gegenüber der Knochenplatte und damit eine Kompression bewirken zu können. Diese grossen Kräfte in der Eindrehrichtung können aber bereits zu einer unnötigen Beschädir gung des Schraubenkopfes, der Knochenplatte oder sogar des Knochens führen. Zudem besteht aufgrund der grossen erforderlichen Kräfte die Gefahr, dass das zum Eindrehen verwendete Werkzeug versehentlich abrutscht. Mit anderen Worten besteht also eine ungünstige Kräfteübertragung zwischen der in der Eindrehrichtung wirkenden Kraft und der die Lateralbewegung herzvorrufenden Kraft. Wird hingegen der Winkel zwischen Gleitfläche und Plattenebene zu steil gewählt, so muss die Platte bei vorgegebener Länge des Kompressionsweges zumindest im Bereich des Kompressionslochs entsprechend dicker ausgebildet sein. Dies führt nicht nur zu einem erhöhten Materialbedarf, sondern kann dem umgebenden Körpergewebe ebenfalls unnötigen Schaden zufügen.

Ferner weisen zwar die in US 7,354,441 B2, WO 00/66012 A1, WO 2010/115403 A1 und EP 2 364 658 A1 offenbarten Kompressionslöcher Gewinde auf, die eine Verblockung eines entsprechend ausgebildeten Schraubenkopfes ermöglichen. Allerdings geben derartige Gewinde eine einzige Verblockungsrichtung vor, in der die Knochenschraube ausschliesslich verblockt werden kann; eine Verblockung unter verschiedenen Winkeln relativ zur Knochenplatte ist also nicht möglich.

Zur Erzielung einer Kompression sind aus dem Stand der Technik auch noch weitere Mechanismen bekannt, die jedoch keine Verblockung erlauben oder weitere Nachteile aufweisen:
So offenbart die WO 2014/033088 A1 eine zweiteilige Knochenplatte mit einem Hauptkörper und einem beweglichen Teil. Das bewegliche Teil verfügt über eine Zahnstange. Durch Rotation eines Zahnrades kann dieses derart mit der Zahnstange zusammenwirken, dass die beiden Teile relativ zueinander verschoben werden, wodurch eine Kompression erreicht werden kann. Das Eindrehen einer Knochenschraube führt aber nicht automatisch zu einer Kompression der zu verbindenden Knochenfragmente. Zudem kann nicht unmittelbar eine Verblockung erreicht werden, geschweige denn eine unter verschiedenen Winkeln relativ zur Knochenplatte.

Die US 2012/0197303 A1 befasst sich mit Knochenplatten mit Langlöchern, an deren einer Seite eine Zahnstange angeordnet ist. In ein solches Langloch kann eine Ritzelwellenanordnung eingesetzt werden. Beim Eindrehen eines Gewindeendes der Ritzelwellenanordnung in einen Knochen erfolgt durch Zusammenwirken des Ritzels mit der Zahnstange eine Kompression. Allerdings enthalten die Schraubenöffnungen dieser Knochenplatten keine Konturen, die sowohl eine Kompression als auch eine Verblockung ermöglichen, geschweige denn eine Verblockung unter verschiedenen Winkeln relativ zur Knochenplatte.

Die US 2009/0234359 A1 offenbart eine Knochenplatte mit einem Langloch, welches über eine Zahnstange verfügt. In dieses Langloch kann ein Ritzel eingesetzt werden, durch dessen Drehung eine Kompression herbeigeführt werden kann. Wie bei der US 2012/0197303 A1 sind aber auch hier keine Schraubenöffnungen mit Konturen vorhanden, die sowohl eine Kompression als auch eine Verblockung ermöglichen, geschweige denn eine Verblockung unter verschiedenen Winkeln relativ zur Knochenplatte.

Es ist daher eine Aufgabe der vorliegenden Erfindung, eine Knochenplatte bereitzustellen, die die oben erläuterten Nachteile des Standes der Technik nicht aufweist. Insbesondere soll die Knochenplatte mindestens eine Aufnahmeöffnung für eine Knochenschraube aufweisen, die sowohl eine Kompression als auch eine Verblockung erlaubt. Dabei soll während der Kompression eine möglichst günstige Kräfteübertragung erzielt werden, ohne dass die Knochenplatte nur zu diesem Zweck im Bereich der Aufnahmeöffnung eine übermässige Dicke aufweisen muss. Bevorzugt soll die Aufnahmeöffnung eine Verblockung unter verschiedenen Winkeln relativ zur Knochenplatte erlauben.

Diese und weitere Aufgaben werden zum einen durch eine erfindungsgemässe Knochenplatte gelöst. Diese Knochenplatte weist mindestens eine Aufnahmeöffnung für eine Knochenschraube mit einem Schraubenkopf auf. Die Aufnahmeöffnung enthält einen Kompressionsbereich mit einer Kompressionskontur und einen Verblockungsbereich mit einer Verblockungskontur. Die Kompressionskontur ist derart ausgebildet und angeordnet, dass eine Rotation der Knochenschraube in einer Eindrehrichtung eine Lateralbewegung der Knochenschraube in Richtung des Verblockungsbereiches bewirkt.

Erfindungsgemäss ist die Kompressionskontur als Eingriffskontur ausgebildet, so dass die Lateralbewegung zumindest teilweise, insbesondere vollständig, durch einen Eingriff zwischen der Eingriffskontur und einer Gegenkontur am Schraubenkopf erzeugbar ist.

Dass die Lateralbewegung "zumindest teilweise" durch einen Eingriff zwischen der Eingriffskontur und einer Gegenkontur am Schraubenkopf erzeugbar ist, bedeutet hierbei, dass dieser Eingriff während zumindest eines Teils des Kompressionsvorganges vorliegt. Dies schliesst also nicht aus, dass während eines anderen Teils des Kompressionsvorganges kein solcher Eingriff vorliegt oder die Lateralbewegung jedenfalls nicht hierdurch erzeugt wird.

Im Gegensatz zum Stand der Technik wird die Lateralbewegung also nicht ausschliesslich durch ein Heruntergleiten der Unterseite des Schraubenkopfes an einer Gleitfläche der Kompressionskontur erzeugt, die durch die axiale Bewegung der Knochenschraube hervorgerufen wird. Stattdessen sorgt die Rotation der Knochenschraube in ihrer Eindrehrichtung durch einen Eingriff zwischen Eingriffskontur und Gegenkontur zumindest teilweise oder sogar vollständig für diese Lateralbewegung. Auf diese Weise kann eine vorteilhafte Kräfteübertragung erzielt werden, ohne dass die Knochenplatte im Bereich der Aufnahmeöffnung zu diesem Zweck eine übermässige Dicke aufweisen muss.

Insbesondere kann in einzelnen Ausführungsformen vollständig auf die aus dem Stand der Technik bekannten, gegenüber der Plattenebene geneigten Gleitflächen verzichtet werden. Die Dicke der Knochenplatte im Bereich der Aufnahmeöffnung wird nicht allein durch die Steigung einer solchen Gleitfläche gegenüber der Plattenebene und durch die Länge des Kompressionsweges bestimmt.

Sowohl der Kompressionsbereich als auch der Verblockungsbereich können eine jeweilige Hauptachse aufweisen. Eine solche Hauptachse des Kompressionsbereiches hat die Eigenschaft, dass eine Knochenschraube zumindest derart in den Kompressionsbereich einsetzbar ist, dass ihre Längsachse parallel zu dieser Hauptachse verläuft. Es ist denkbar, dass die Knochenschraube zusätzlich derart in den Kompressionsbereich einsetzbar ist, dass ihre Längsachse innerhalb eines die Hauptachse des Kompressionsbereiches enthaltenden Raumwinkelbereiches verläuft. In diesem Falle ist die genannte Hauptachse nicht eindeutig definiert, d. h. mehrere voneinander verschiedene Achsen können als Hauptachse im Sinne der obigen Definition verstanden werden.

Analog dazu hat eine Hauptachse des Verblockungsbereiches die Eigenschaft, dass eine Knochenschraube zumindest derart in den Verblockungsbereich einsetzbar ist, dass ihre Längsachse parallel zu dieser Hauptachse verläuft. Es ist denkbar, dass die Knochenschraube zusätzlich derart in den Verblockungsbereich einsetzbar ist, dass ihre Längsachse innerhalb eines die Hauptachse des Verblockungsbereiches enthaltenden Raumwinkelbereiches verläuft. In diesem Falle ist die genannte Hauptachse nicht eindeutig definiert, d. h. mehrere voneinander verschiedene Achsen können als Hauptachse im Sinne der obigen Definition verstanden werden. Die Hauptachse des Kompressionsbereiches und die Hauptachse des Verblockungsbereiches können sich unabhängig voneinander senkrecht oder geneigt zur Plattenebene erstrecken. Die Hauptachse des Kompressionsbereiches und die Hauptachse des Verblockungsbereiches können voneinander einen Abstand aufweisen, der im Bereich von 0,2 mm bis 5 mm, bevorzugt von 0,5 mm bis 2 mm, besonders bevorzugt von 0,8 mm bis 1,2 mm liegt.

Vorteilhafterweise ist die Lateralbewegung zumindest teilweise, insbesondere vollständig, durch ein Abwälzen des Schraubenkopfes entlang eines Teils der Eingriffskontur erzeugbar, insbesondere entlang eines weiter unten noch beschriebenen Abwälzbereiches. Unter einem Abwälzen wird hier und im Folgenden verstanden, dass der Schraubenkopf an einem Teil der Eingriffskontur abrollt, wobei dieses Abrollen von einem Gleiten überlagert sein kann. Dieses Abwälzen erfolgt also nach dem Prinzip einer Verzahnung zwischen zwei Zahnrädern oder einem Zahnrad und einer Zahnstange. Ein solches Abwälzen ermöglicht eine präzise Führung des Schraubenkopfes und reduziert den Materialabrieb, der beim reinen Gleiten aufträte. Ebenfalls ist ein Anpressen der Knochenschraube auf die Knochenplatte als Bedingung für die Lateralbewegung praktisch nicht erforderlich. Des Weiteren kann die Bewegung auf theoretisch unbegrenzt langen und/oder auf nicht geraden Bahnen erfolgen.

Dass die Lateralbewegung "zumindest teilweise" durch ein Abwälzen erzeugbar ist, bedeutet hierbei, dass die Lateralbewegung während zumindest eines Teils des Kompressionsvorganges durch ein Abwälzen erzeugbar ist und/oder nur ein Abwälzen zwischen einzelnen Teilen der Eingriffskontur und/oder der Gegenkontur erzeugbar ist. So ist es beispielsweise denkbar und von der Formulierung "zumindest teilweise" erfasst, dass während eines anderen Teils des Kompressionsvorganges die Lateralbewegung nicht durch ein Abwälzen erzeugbar ist. Von der Formulierung "zumindest teilweise" ist ebenfalls erfasst, dass ein erster Teil des Schraubenkopfes an einem ersten Teil der Eingriffskontur abgewälzt wird, während gleichzeitig ein zweiter Teil des Schraubenkopfes an einem zweiten Teil der Eingriffskontur entlang gleitet.

Bevorzugt weist die Eingriffskontur mindestens eine Führungsfläche auf, entlang welcher während der Rotation der Knochenschraube in ihrer Eindrehrichtung eine Unterseite des Schraubenkopfes führbar ist. Eine solche Führung erlaubt eine präzisiere Bewegung des Schraubenkopfes. Sie kann ebenfalls zur Erzeugung der Lateralbewegung beitragen, wobei die Lateralbewegung erfindungsgemäss jedoch zumindest teilweise auch oder sogar nur durch den Eingriff zwischen Eingriffskontur und Gegenkontur erzeugt wird. Weiterhin sorgt die genannte Führung dafür, dass der Schraubenkopf die Knochenplatte in Richtung auf das Knochenfragment zieht, in welches sie eingedreht wird.

Die Führungsfläche ist bevorzugt zykloidenartig ausgebildet. Unter einer zykloidenartigen Ausbildung wird hier und im Folgenden eine derartige Beschaffenheit der Führungsfläche verstanden, die es ermöglicht, dass die Knochenschraube, insbesondere eine Unterseite eines Verblockungsvorsprunges der Knochenschraube, zumindest während eines Teiles der resultierenden Bewegung, die aus der helixförmigen Bewegung der Knochenschraube um ihre Längsachse und der Bewegung in Richtung des Verblockungsbereiches resultiert, in Kontakt mit der Führungsfläche verbleibt. Hierdurch kann die Führungsfläche die genannte resultierende Bewegung besonders gut führen.

Ebenfalls bevorzugt ist die Führungsfläche so ausgestaltet, dass entlang dieser Führungsfläche während der Rotation der Knochenschraube in ihrer Eindrehrichtung eine Unterseite eines radial am Schraubenkopf angeordneten Verblockungsvorsprunges führbar ist. Ein solcher Verblockungsvorsprung kann der Verblockung des Schraubenkopfes an der Verblockungskontur dienen, wie weiter unten noch im Detail erläutert wird. Der Verblockungsvorsprung kann also sowohl für die Führung des Schraubenkopfes während der Kompression sorgen als auch für die anschliessende Verblockung; er kann also eine doppelte Funktion übernehmen.

Die Führung erfolgt bevorzugt entlang einer Längsachse der Knochenschraube, also in der Richtung, in der die Knochenschraube in den Knochen eingedreht wird.

Die Eingriffskontur kann mindestens einen Abwälzbereich aufweisen, an dem ein radial am Schraubenkopf angeordneter Verblockungsvorsprung während zumindest eines Teils der Lateralbewegung abwälzbar ist.

Der Abwälzbereich kann eine Bodenfläche und eine zwischen der Oberseite und der Bodenfläche verlaufende Seitenfläche enthalten, wobei der genannte radial am Schraubenkopf angeordnete Verblockungsvorsprung während zumindest eines Teils der Lateralbewegung an dieser Seitfläche abwälzbar ist. Die Bodenfläche kann parallel zu einer Oberseite der Knochenplatte verlaufen. Alternativ ist es aber auch denkbar und liegt im Rahmen der Erfindung, dass die Bodenfläche im obigen Sinne zykloidenartig ausgebildet ist.

Mit besonderem Vorteil ist die Verblockungskontur derart ausgebildet, dass die Knochenschraube unter verschiedenen Winkeln relativ zur Knochenplatte verblockbar ist. Wie oben erläutert wurde, muss die Hauptachse des Verblockungsbereiches nicht zwingend eindeutig definiert sein. Die Knochenschraube ist also nicht nur derart im Verblockungsbereich verblockbar, dass ihre Längsachse parallel zu einer einzigen möglichen Hauptachse des Verblockungsbereiches verläuft, sondern auch in davon abweichenden Ausrichtungen. Dies hat den bereits oben erläuterten Vorteil, dass der individuellen Anatomie des behandelten Patienten Rechnung getragen werden kann.

Der Verblockungsbereich und insbesondere dessen Verblockungskontur kann eines, mehrere oder sämtliche der in WO 2004/086990 A1 offenbarten Merkmale aufweisen.

Insbesondere kann die Verblockungskontur mindestens eine Ausnehmung aufweisen, die sich keilförmig nach aussen von einer Hauptachse des Verblockungsbereiches weg erweitert, um einen radial am Schraubenkopf angeordneten Verblockungsvorsprung aufnehmen und verblocken zu können. Diese keilförmige Ausbildung erlaubt ein radiales Einklemmen des Schraubenkopfes, insbesondere von am Schraubenkopf angeordneten Verblockungsvorsprüngen, wenn die Knochenschraube um ihre Längsachse gedreht wird. Vorteilhafterweise ist die Verblockungskontur wenigstens im Bereich der Ausnehmung zumindest annähernd sphärisch, paraboloidisch, ellipsoidisch oder hyperboloidisch ausgebildet. Insbesondere wenn die Verblockungskontur im Bereich der Ausnehmung zumindest annähernd sphärisch ausgebildet ist, ermöglich dies eine Verblockung der Knochenschraube unter verschiedenen Winkeln relativ zur Knochenplatte.

Weiterhin ist die Ausnehmung der Verblockungskontur bevorzugt so angeordnet, dass sie in einer Richtung senkrecht zur Hauptachse des Verblockungsbereiches umläuft.

Die Verblockungskontur kann mindestens zwei, bevorzugt mindestens drei und besonderes bevorzugt genau drei Ausnehmungen aufweisen, die sich jeweils keilförmig nach aussen von der Hauptachse des Verblockungsbereiches weg erweitern. Hierdurch kann der Schraubenkopf besonders stabil in der Verblockungskontur verblockt werden. Dies gilt insbesondere dann, wenn die Ausnehmungen gleichmässig in Umfangsrichtung um die Hauptachse des Verblockungsbereiches herum erstrecken.

Die Verblockungskontur kann im Bereich der sich keilförmig nach aussen von der Hauptachse des Verblockungsbereiches weg erweiternden Ausnehmung in einer senkrecht zur Hauptachse des Verblockungsbereiches verlaufenen Azimutebene durch einen Teil einer logarithmischen Spirale, einer Kreisbahn, einer Evolvente oder einer Wurzelfunktion vom Typ r = a₁ + b₁√α beschrieben werden, wobei r den jeweiligen Abstand der Verblockungskontur von der Hauptachse des Verblockungsbereiches bedeutet, a₁ und b₁ Konstanten sind und α für den jeweiligen Umlaufwinkel steht.

Die Gegenkontur kann durch mindestens einen der Verblockungsvorsprünge und/oder mindestens eine der zwischen den Verblockungsvorsprüngen angeordneten Ausnehmungen gebildet sein. Dann nämlich können die Verblockungsvorsprünge und/oder die Ausnehmungen sowohl der Erzeugung der Lateralbewegung während der Kompression dienen als auch der anschliessenden Verblockung; sie können also eine doppelte Funktion übernehmen.

In vorteilhaften Ausführungsformen verläuft eine Längsachse der Knochenschraube während der Lateralbewegung in Richtung des Verblockungsbereiches entlang einer geradlinigen Bahn. Allerdings ist es natürlich auch denkbar und liegt im Rahmen der Erfindung, dass diese Bahn zumindest abschnittsweise oder sogar vollständig kurvenförmig ist.

Ein weiterer Aspekt der Erfindung betrifft ein Operationsset, welches mindestens eine wie oben offenbarte erfindungsgemässe Knochenplatte enthält sowie mindestens eine Knochenschraube mit einem Schraubenkopf, welche in mindestens einer der Aufnahmeöffnungen der Knochenplatte einsetzbar ist. Auf diese Weise kann die Knochenschraube in der bereits ausführlich erläuterten Weise zusammen mit der Knochenplatte verwendet werden, um die ebenfalls erläuterten Vorteile zu bewirken.

Bei der Knochenschraube kann es sich um eine an sich bekannte Knochenschraube handeln, sofern sie die oben beschriebenen Eigenschaften aufweist, um mit der Knochenplatte in der beschriebenen Weise zusammenwirken zu können.

Bevorzugt weist die Knochenschraube eines, mehrere oder sämtliche der in WO 2004/086990 A1 offenbarten Merkmale auf. Bevorzugt ist der Schraubenkopf mit einem radial am Schraubenkopf angeordneten Verblockungsvorsprung mit einer umlaufenden Aussenfläche versehen, die sich im Wesentlichen in Richtung einer Längsachse der Knochenschraube erstreckt und mindestens eine Klemmfläche aufweist, welche sich, in einer Azimutebene senkrecht zur Längsachse betrachtet, keilförmig nach aussen von der Längsachse weg erweitert, um den Schraubenkopf mit der Verblockungskontur, insbesondere mit deren Ausnehmungen, verblocken zu können.

Ebenfalls mit Vorteil ist die umlaufende Aussenfläche des Schraubenkopfes wenigstens im Bereich der Klemmfläche zumindest annähernd sphärisch, paraboloidisch, ellipsoidisch oder hyperboloidisch ausgebildet.

Ausserdem ist es vorteilhaft, wenn die umlaufende Aussenfläche des Schraubenkopfes mindestens drei, bevorzugt genau drei gleichmässig entlang ihres Umfangs verteilte Klemmflächen aufweist, die sich jeweils keilförmig nach aussen von der Längsachse der Knochenschraube weg erweitern.

Die Kontur der Aussenfläche des Schraubenkopfes im Bereich der sich keilförmig nach aussen von der Längsachse weg erweiternden Klemmfläche kann in eine Azimutebene durch einen Teil einer logarithmischen Spirale, durch einen Teil einer Kreisbahn oder durch einen Teil einer Evolvente oder einer Wurzelfunktion vom Typ r = a₁ + b₁√α beschrieben sein, wobei r den jeweiligen Abstand der Kontur von der Längsachse bedeutet, a₁ und b₁ Konstanten sind und α für den jeweiligen Umlaufwinkel steht.

Der Verblockungsbereich kann mit einer insbesondere sphärischen Ansenkung versehen sein zur Aufnahme beispielsweise eines Schraubenkopfs mit sphärischer Kopfunterseite.

Enthält die Knochenplatte eine wie oben beschriebene Führungsfläche, so ist diese bevorzugt derart auf die Knochenschraube abgestimmt, insbesondere auf eine Unterseite des Schraubenkopfes und eine Gewindesteigung eines Schaftes der Knochenschraube, dass die Unterseite des Schraubenkopfes, insbesondere eine Unterseite eines radial am Schraubenkopf angeordneten Verblockungsvorsprunges, während des Eindrehens der Knochenschraube entlang der Führungsfläche führbar ist, insbesondere entlang einer Längsachse der Knochenschraube. Die Vorteile einer solchen Führung wurden bereits oben hervorgehoben.

Enthält mindestens eine Knochenplatte einen wie oben beschriebenen Abwälzbereich, so ist bevorzugt dieser Abwälzbereich, insbesondere dessen Seitenfläche, derart auf die Knochenschraube abgestimmt, insbesondere auf eine am Schraubenkopf angeordnete Gegenkontur und eine Gewindesteigung eines Schaftes der Knochenschraube, dass der Schraubenkopf während des Eindrehens der Knochenschraube am Abwälzbereich, insbesondere an dessen Seitenfläche, abwälzbar ist.

Weiterhin wird hierin ein Verfahren zum Fixieren einer wie oben beschriebenen erfindungsgemässen Knochenplatte an zwei zu verbindenden Knochenfragmenten beschrieben. Dieses Verfahren enthält die Schritte:
a) Fixieren der Knochenplatte an einem ersten Knochenfragment mithilfe einer ersten Knochenschraube,
b) Einsetzen einer zweiten Knochenschraube durch den Kompressionsbereich der Aufnahmeöffnung hindurch in ein zweites Knochenfragment,
c) Rotation der zweiten Knochenschrauben in einer Eindrehrichtung, wodurch eine Lateralbewegung der zweiten Knochenschraube in Richtung des Verblockungsbereiches bewirkt wird und die Lateralbewegung zumindest teilweise, insbesondere vollständig, durch einen Eingriff zwischen der Eingriffskontur und einer Gegenkontur am Schraubenkopf erzeugt wird.

In diesem Verfahren werden also die oben erläuterten vorteilhaften strukturellen Eigenschaften der erfindungsgemässen Knochenplatte ausgenutzt. Die zweite Knochenschraube kann eines, mehrere oder sämtliche der oben im Zusammenhang mit dem erfindungsgemässen Operationsset beschriebenen Merkmale aufweisen.

Im Schritt a) kann die Knochenschraube durch eine Aufnahmeöffnung der Knochenplatte hindurch in das erste Knochenfragment eingedreht werden. Diese Aufnahmeöffnung kann die erfindungsgemässen Eingriffs- und Verblockungskonturen aufweisen. Es kann sich jedoch auch um eine aus dem Stand der Technik bekannte Aufnahmeöffnung handeln, beispielsweise um eine wie in WO 2004/086990 A1 beschriebene oder eine rein zylindrische.

Optional kann das Verfahren noch den nachfolgenden weiteren Schritt enthalten:
d) Verblocken der zweiten Knochenschraube in der Verblockungskontur.

Im Folgenden wird die Erfindung anhand eines Ausführungsbeispiels und mehrerer Zeichnungen erläutert. Dabei zeigen
- Figur 1a:: eine erfindungsgemässe Knochenplatte in Form einer Condylusplatte in einer Draufsicht;
- Figur 1b:: die Knochenplatte gemäss Figur 1a in einer perspektivischen Ansicht;
- Figur 2a:: eine Detailansicht einer der Aufnahmeöffnungen der Knochenplatte in einer ersten perspektivischen Ansicht;
- Figur 2b:: die Detailansicht gemäss Figur 2a in einer zweiten perspektivischen Ansicht;
- Figur 3a:: die Detailansicht gemäss Figuren 2a und b in einer Draufsicht;
- Figur 3b:: eine Schnittansicht entlang der Linie C-C in Figur 3a;
- Figur 3c:: eine Schnittansicht entlang der Linie D-D in Figur 3a;
- Figur 4a:: eine Knochenschraube in einer perspektivischen Ansicht;
- Figur 4b:: die Knochenschraube gemäss Figur 4a in einer Draufsicht;
- Figur 5a:: die Detailansicht gemäss Figur 2a mit einer in einem Kompressionsbereich der Aufnahmeöffnung eingesetzten Knochenschraube gemäss Figuren 4a und b in einer ersten perspektivischen Ansicht;
- Figur 5b:: die Detailansicht gemäss Figur 5a in einer zweiten perspektivischen Ansicht;
- Figur 6a:: die Detailansicht gemäss Figuren 5a und b in einer Draufsicht;
- Figur 6b:: eine Schnittansicht entlang der Linie E-E in Figur 6a (wobei jedoch die Knochenschraube nicht geschnitten dargestellt ist);
- Figur 6c:: eine Schnittansicht entlang der Linie F-F in Figur 6a (wobei jedoch die Knochenschraube nicht geschnitten dargestellt ist);
- Figur 7a:: die Detailansicht gemäss Figur 2a mit der Knochenschraube im Übergangsbereich zwischen Kompressionsbereich und Verblockungsbereich in einer ersten perspektivischen Ansicht;
- Figur 7b:: die Detailansicht gemäss Figur 7a in einer zweiten perspektivischen Ansicht;
- Figur 8a:: die Detailansicht gemäss Figuren 7a und b in einer Draufsicht;
- Figur 8b:: eine Schnittansicht entlang der Linie G-G in Figur 8a (wobei jedoch die Knochenschraube nicht geschnitten dargestellt ist);
- Figur 8c:: eine Schnittansicht entlang der Linie H-H in Figur 8a (wobei jedoch die Knochenschraube nicht geschnitten dargestellt ist);
- Figur 9a:: die Detailansicht gemäss Figur 2a mit der Knochenschraube im Verblockungsbereich in einer ersten perspektivischen Ansicht;
- Figur 9b:: die Detailansicht gemäss Figur 9a in einer zweiten perspektivischen Ansicht;
- Figur 10a:: die Detailansicht gemäss Figuren 9a und b in einer Draufsicht;
- Figur 10b:: eine Schnittansicht entlang der Linie I-I in Figur 10a (wobei jedoch die Knochenschraube nicht geschnitten dargestellt ist);
- Figur 10c:: eine Schnittansicht entlang der Linie J-J in Figur 10a (wobei jedoch die Knochenschraube nicht geschnitten dargestellt ist).

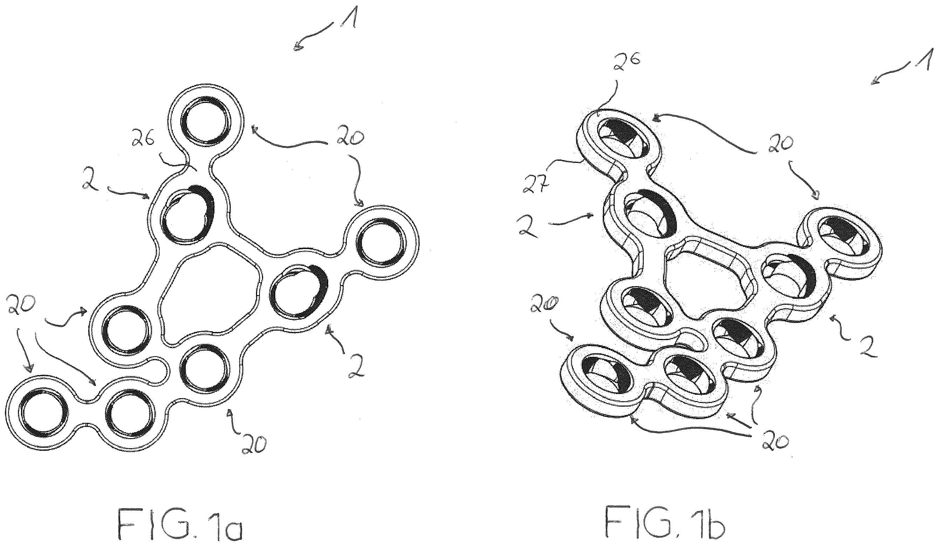

Die Figuren 1a und 1b zeigen in zwei Ansichten eine als Condylusplatte ausgebildete Knochenplatte 1. Diese enthält eine Oberseite 26, eine dazu parallel verlaufende Unterseite 27 und zwei weiter unten im Detail beschriebene erfindungsgemässe Aufnahmeöffnungen 2 und sechs nicht erfindungsgemässen Aufnahmeöffnungen 20, die sich allesamt von der Oberseite 26 zur Unterseite 27 erstrecken. Die nicht erfindungsgemässen Aufnahmeöffnungen 20 können beispielsweise eine wie in WO 2004/086990 A1 offenbarte Verblockungskontur aufweisen.

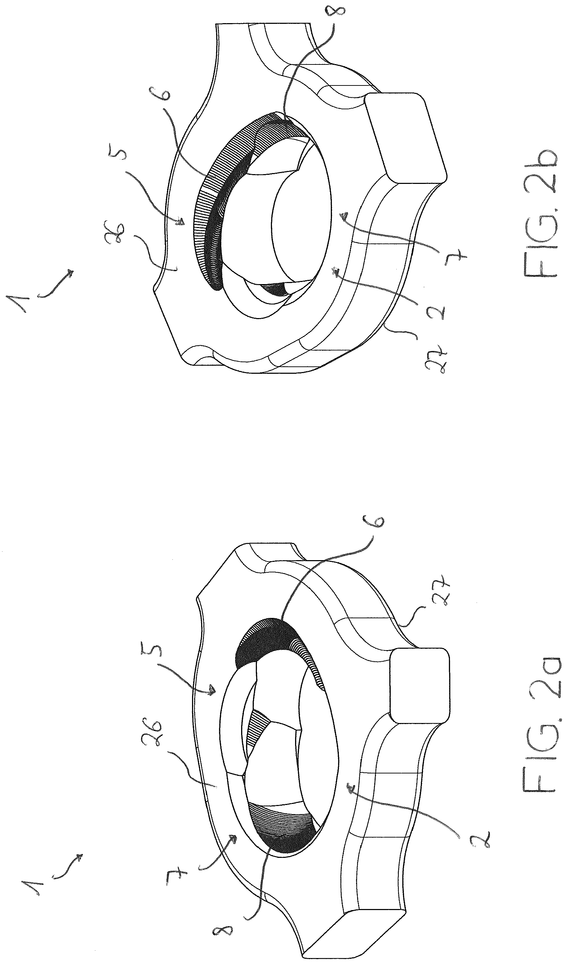

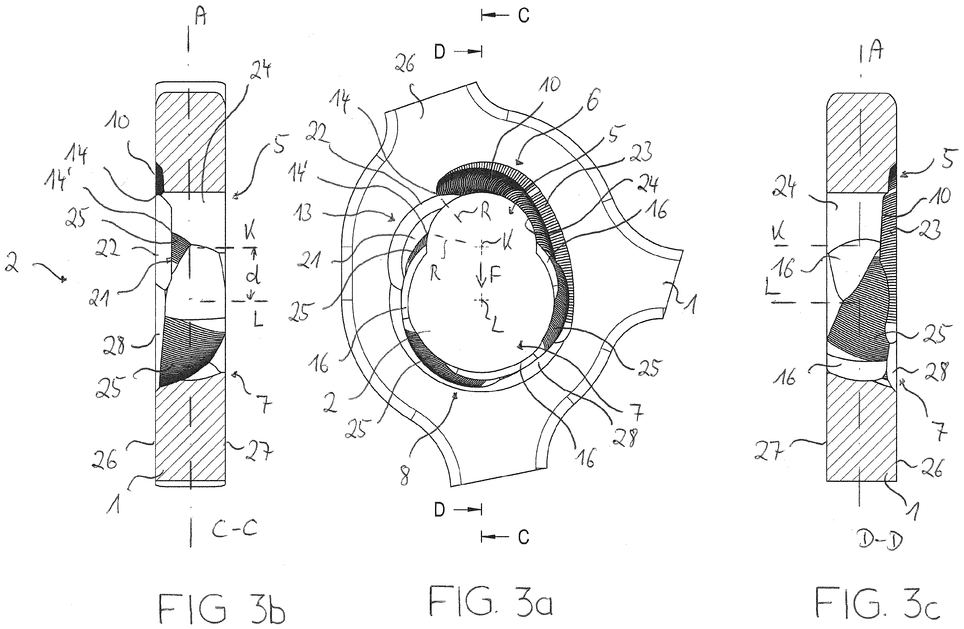

Die Figuren 2a, 2b und 3a bis 3c zeigen eine der beiden in Figur 1 erkennbaren Aufnahmeöffnungen 2 in zwei verschiedenen perspektivischen Ansichten, einer Draufsicht und zwei Schnittansichten. Die Aufnahmeöffnung 2 enthält einen Kompressionsbereich 5 mit einer als Eingriffskontur 6 ausgebildeten Kompressionskontur und einen Verblockungsbereich 7 mit einer Verblockungskontur 8. Der Kompressionsbereich 5 und der Verblockungsbereich 7 weisen jeweils eine kreisförmige Silhouette auf, wobei diese beiden kreisförmigen Silhouetten einander durchdringen. Durch den Mittelpunkt der kreisförmigen Silhouette des Kompressionsbereiches 5 und senkrecht zur Oberseite 26 der Knochenplatte 1 verläuft eine Hauptachse K des Kompressionsbereiches 5. Der Kompressionsbereich wird teilweise durch einen zylindrischen Wandabschnitt 24 begrenzt. Durch den Mittelpunkt der kreisförmigen Silhouette des Verblockungsbereiches 7 und ebenfalls senkrecht zur Oberseite 26 der Knochenplatte 1 verläuft eine Hauptachse L des Verblockungsbereiches 7. Die Hauptachse K des Kompressionsbereiches 5 und die Hauptachse L des Verblockungsbereiches 7 haben im hier gezeigten Ausführungsbeispiel voneinander einen Abstand d=1 mm.

Die Eingriffskontur 6 weist eine zykloidenartige Führungsfläche 10 auf. Zwischen der Führungsfläche 10 und einer Oberseite 26 der Knochenplatte 1 ist eine geneigt verlaufende Seitenwand 23 gebildet. Die Eingriffskontur 6 enthält ferner einen Abwälzbereich 13, der eine parallel zur Oberseite 26 verlaufende Bodenfläche 21 und eine zwischen der Oberseite 26 und der Bodenfläche 21 verlaufende Seitenfläche 22 enthält.

Zudem weist die Eingriffskontur 6 zwei sich in einer radialen Richtung R erstreckende Vorsprünge 14, 14' auf. Die radiale Richtung R wird dabei bezüglich der Hauptachse K des Kompressionsbereiches 5 verstanden. Der erste Vorsprung 14 ist zwischen der Führungsfläche 10 und dem Abwälzbereich 13 angeordnet - genauer: am Berührungspunkt der Oberseite 26 der Knochenplatte 1, der Führungsfläche 10 und der Seitenfläche 22 des Abwälzbereiches 13. Der zweite Vorsprung 14' ist zwischen dem Abwälzbereich 13 und der Verblockungskontur 8 angeordnet - genauer: am Berührungspunkt der Bodenfläche 21 des Abwälzbereiches 13, des zylindrischen Wandabschnittes 24 und einer der drei unten noch erläuterten Ausnehmungen 16.

Die Verblockungskontur 8 weist drei Ausnehmungen 16 auf, von denen zwei durch den Kompressionsbereich 5 unterbrochen sind. Die drei Ausnehmungen 16 erweitern sich keilförmig nach aussen von einer Hauptachse L des Verblockungsbereiches 7 weg. Im Bereich dieser Ausnehmungen 16 ist die Verblockungskontur 8 jeweils sphärisch ausgebildet. Alternativ können die Ausnehmungen 16 aber beispielsweise auch paraboloidisch, ellipsoidisch oder hyperboloidisch ausgebildet sein. Die Ausnehmungen 16 sind ferner so angeordnet, dass sie in einer Richtung senkrecht zur Hauptachse L des Verblockungsbereiches 7 umlaufen. Im Bereich der Ausnehmungen 16 wird die Verblockungskontur 8 in einer senkrecht zur Hauptachse L der Verblockungsbereiches 7 verlaufenden Azimutebene A jeweils durch eine Wurzelfunktion beschrieben, also eine Funktion vom Typ r = a₁ + b₁√α, wobei r den jeweiligen Abstand der Verblockungskontur 8 von der Hauptachse L des Verblockungsbereiches 7 bedeutet, a₁ und b₁ Konstanten sind und α für den jeweiligen Umlaufwinkel steht. Die Azimutebene A entspricht der Zeichenebene der Figur 3a.

An jede der drei Ausnehmungen 16 schliesst sich jeweils eine Auslaufkontur 25 an, welche dem Ausleiten einer Knochenschraube dient, um sie einfacher wieder aus der Aufnahmeöffnung 2 entnehmen zu können. Zudem ist eine Ansenkung 28 vorhanden, die zur Aufnahme eines Schraubenkopfes mit sphärischer Kopfunterseite dienen kann.

Zur Herstellung der Aufnahmeöffnung 2 kann in einem ersten Schritt zunächst ein zylindrischer Fräser verwendet werden, mit dem die Silhouette des Kompressionsbereichs 5 gefertigt wird. Der zylindrische Wandabschnitt 24 stammt von diesem ersten Schritt. Die weiteren Konturen der Aufnahmeöffnung 2 können anschliessend in einem zweiten Schritt mit Hilfe eines wie in WO 2004/086990 A1 offenbarten Fräsers gefertigt werden, der einen konvexen Fräserkopf mit einer zumindest annähernd sphärisch ausgebildeten Kontur enthält.

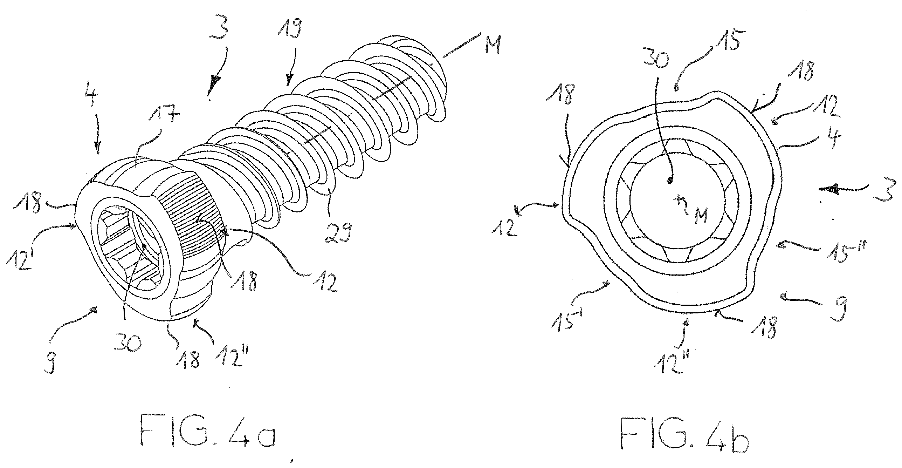

Die Figuren 4a und 4b zeigen eine Knochenschraube 3, welche winkelvariabel in jeder der Aufnahmeöffnungen 2, 20 der Knochenplatte 1 eingesetzt werden kann. Diese Knochenschraube 3 ist identisch zu der in WO 2004/086990 offenbarten. Sie weist einen Schaft 19 mit einem Gewinde 29 sowie einen Schraubenkopf 4 auf, der über den Schaft 19 und das Gewinde 29 nach aussen vorsteht. Der Schraubenkopf 4 weist eine Eingriffskontur 30 auf, in die beispielsweise ein Schraubendreher eingesetzt werden kann, um die Knochenschraube 3 ein- oder auszudrehen. Ferner ist der Schraubenkopf 4 mit einer umlaufenden Aussenfläche 17 versehen, die sich im Wesentlichen in Richtung einer Längsachse M der Knochenschraube 3 erstreckt und drei in Umfangsrichtung gleichmässig verteilte, sich in radialer Richtung erstreckende Verblockungsvorsprünge 12, 12', 12" mit jeweiligen Klemmflächen 18 aufweist. Zwischen den Verblockungsvorsprüngen 12, 12', 12" sind jeweils Ausnehmungen 15, 15', 15" gebildet.

In einer Azimutebene senkrecht zur Längsachse M betrachtet (Zeichenebene der Figur 4b) erweitern sich die Klemmflächen 18 keilförmig nach aussen und von der Längsachse M weg. Die Aussenfläche 17 ist im Bereich der Klemmflächen 18 sphärisch ausgebildet.

Zum einen bilden die Verblockungsvorsprünge 12, 12', 12" und die dazwischen geformten Ausnehmungen 15, 15', 15" eine Gegenkontur 9, die zusammen mit der Eingriffskontur 6 der Knochenplatte 1 eine Lateralbewegung der Knochenschraube 3 bewirken kann. Zum anderen erlauben es diese Klemmflächen 18, den Schraubenkopf 4 mit der Verblockungskontur 8 verblocken zu können, und zwar unter verschiedenen Winkeln relativ zur Knochenplatte, so wie es im Detail in WO 2004/086990 A1 beschrieben ist (welche jedoch lediglich eine Aufnahmeöffnung ohne den erfindungsgemässen Kompressionsbereich offenbart).

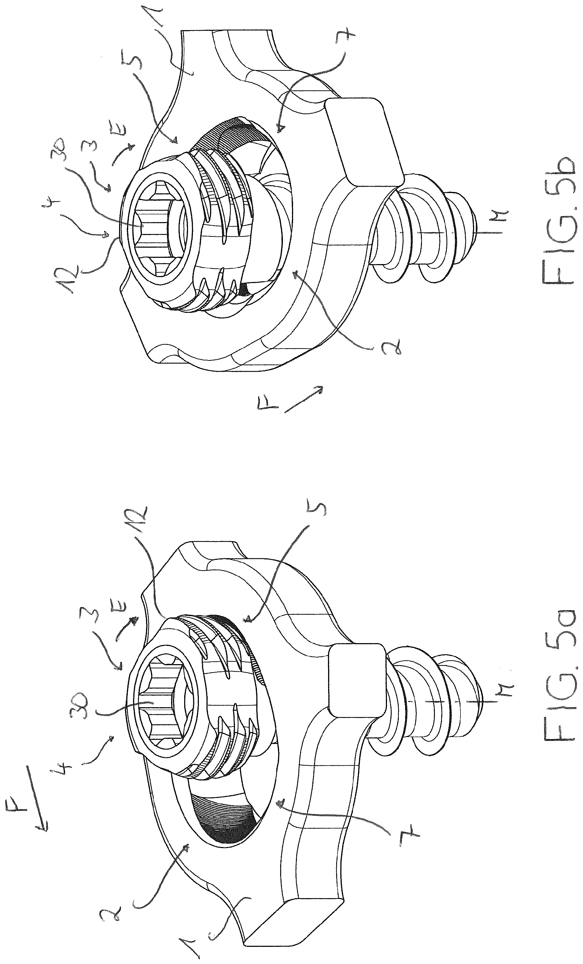

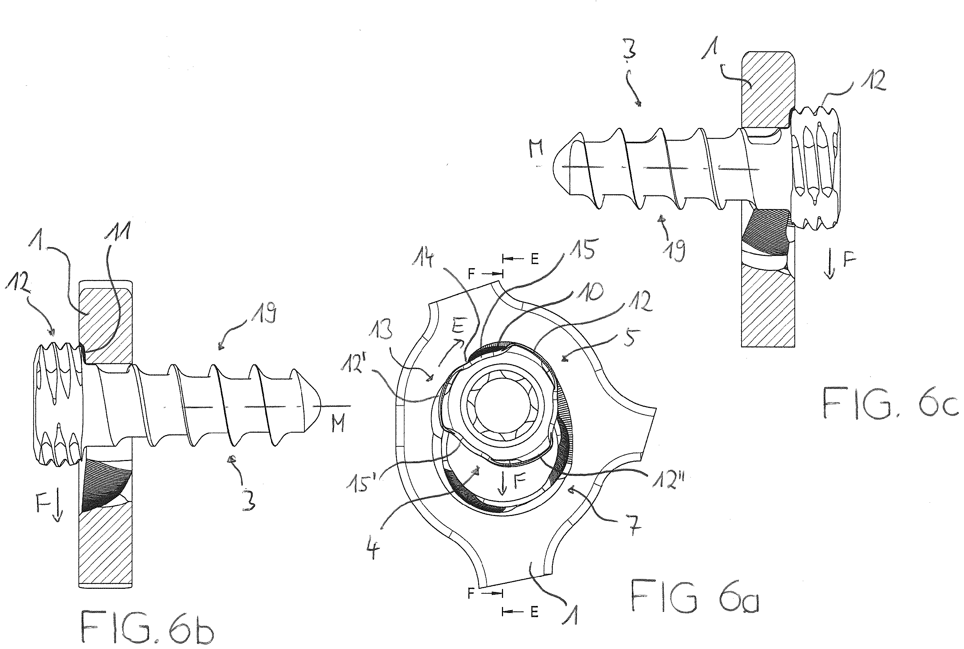

Zum Fixieren der Knochenplatte 1 an zwei zu verbindenden Knochenfragmenten wird die Knochenplatte 1 durch Setzen einer Knochenschraube durch eines der Plattenlöcher 20 in Figur 1a/b zunächst in einem hier nicht dargestellten Schritt a) an einem ersten hier nicht dargestellten Knochenfragment fixiert. In einem anschliessenden Schritt b) wird eine zweite Knochenschraube 3 durch den Kompressionsbereich 5 der Aufnahmeöffnung 2 hindurch in das zweite Knochenfragment eingesetzt. Zu diesem Zeitpunkt nehmen die Knochenschraube 3 und die Aufnahmeöffnung 2 die in den Figuren 5a bis 6c dargestellte Relativposition ein. Der Schaft 19 ist in das hier zur Vereinfachung nicht dargestellte zweite Knochenfragment eingedreht. Eine Unterseite 11 eines ersten Verblockungsvorsprungs 12 liegt an der Führungsfläche 10 und auch an der Seitenwand 23 an.

Daraufhin wird mit Schritt c) begonnen: Mittels eines Schraubendrehers, der in die Eingriffskontur 30 eingreift, wird eine Rotation der Knochenschraube 3 in einer Eindrehrichtung E bewirkt. Die Knochenschraube 3 bewegt sich hierdurch in Richtung ihrer Längsachse M, und der Schaft 19 wird weiter in das zweite Knochenfragment hineingetrieben.

Dabei gleitet zum einen die Unterseite 11 des ersten Verblockungsvorsprunges 12 an der zykloidenartigen Führungsfläche 10 herunter und an der Seitenwand 23 entlang. Um dies zu ermöglichen, ist die Steigung der Führungsfläche 10 auf die Unterseite 11 des Verblockungsvorsprunges 12 und auf die Gewindesteigung des Schaftes 19 abgestimmt. Dies trägt bereits zu einer Lateralbewegung der Knochenschraube 3 in Richtung des Verblockungsbereiches 7, also in der Lateralrichtung F, bei. Relativ zum zweiten Knochenfragment, an dem die zweite Knochenschraube 3 befestigt ist, bewegt sich folglich die Knochenplatte 1 entgegen der Lateralrichtung F.

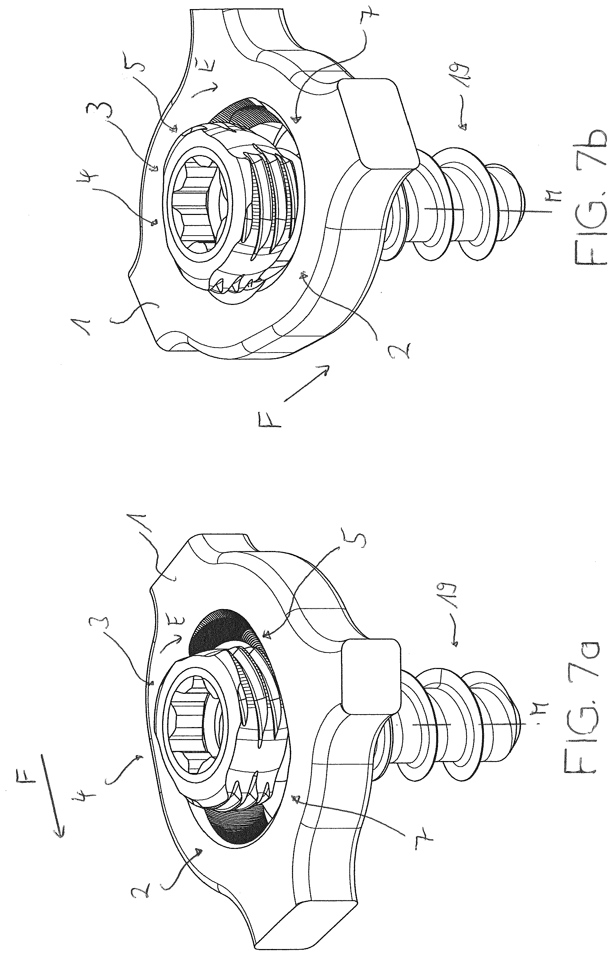

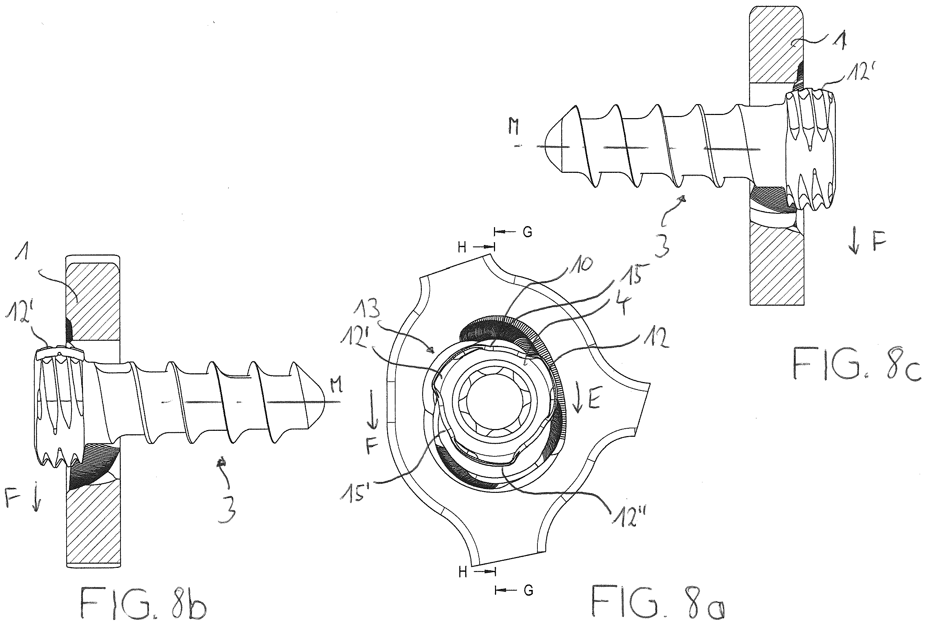

Erfindungsgemäss wird diese Lateralbewegung aber zusätzlich durch einen Eingriff zwischen der Eingriffskontur 6 und der Gegenkontur 9 am Schraubenkopf 4 erzeugt: Während des Überganges in die in den Figuren 7a bis 8c dargestellte Position wird der zweite Verblockungsvorsprung 12' an der Seitenfläche 22 des Abwälzbereiches 13 abgewälzt. Zu diesem Zweck ist die Seitenfläche 22 des Abwälzbereiches 13 entsprechend auf den Verblockungsvorsprung 12' und die Gewindesteigung des Schaftes 19 abgestimmt.

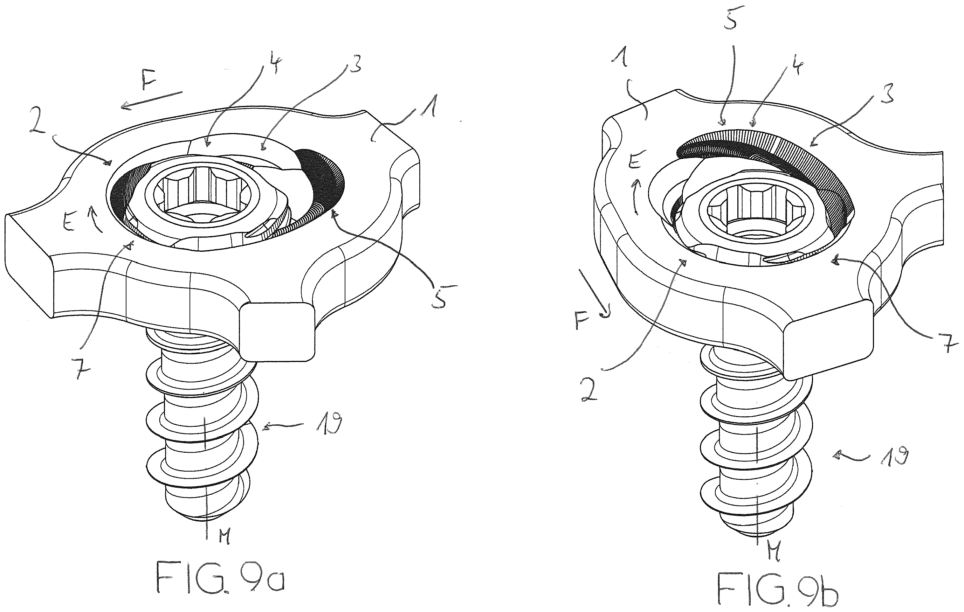

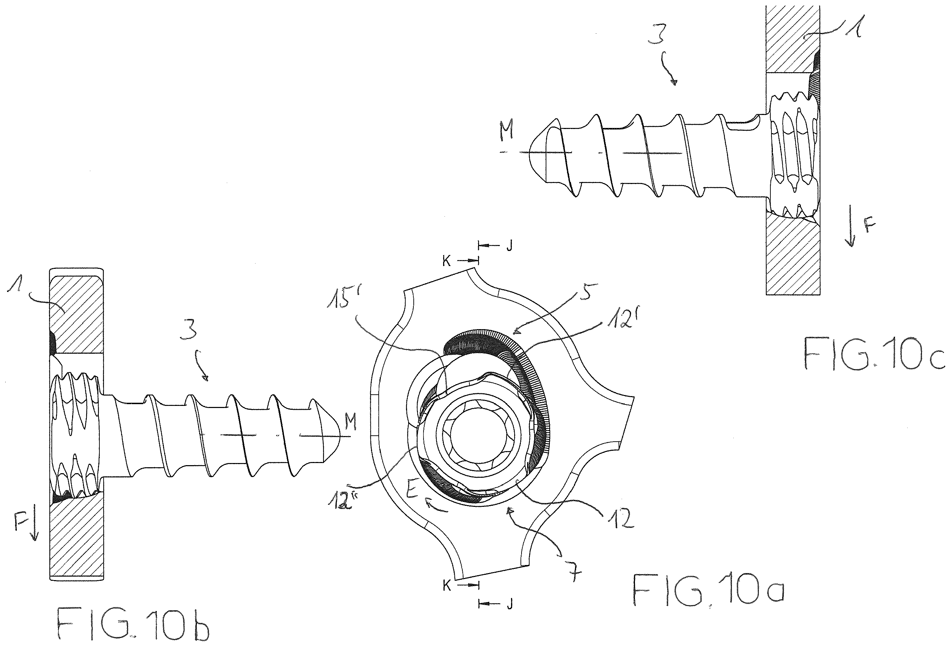

Wird die Knochenschraube 3 dann noch weiter in ihrer Eindrehrichtung E gedreht, so zieht sie sich noch weiter in das zweite Knochenfragment hinein. Schliesslich gelangt die Knochenschraube 3 in die in den Figuren 9a bis 10c dargestellte Position, in der sie sich im Verblockungsbereich 8 der Aufnahmeöffnung 2 befindet. Durch weitere Rotation der Knochenschraube 3 in der Eindrehrichtung E kann in einem Schritt d) eine Verblockung zwischen den Verblockungsvorsprüngen 12 und den Ausnehmungen 16 der Verblockungskontur 8 erreicht werden, so wie es im Detail in der WO 2004/086990 A1 beschrieben ist (welche jedoch lediglich eine Aufnahmeöffnung ohne den erfindungsgemässen Kompressionsbereich offenbart).

## Patentansprüche

1. Knochenplatte (1) mit mindestens einer Aufnahmeöffnung (2) für eine Knochenschraube (3) mit einem Schraubenkopf (4), wobei die Aufnahmeöffnung (2) einen Kompressionsbereich (5) mit einer Kompressionskontur (6) und einen Verblockungsbereich (7) mit einer Verblockungskontur (8) enthält, wobei die Kompressionskontur (6) derart ausgebildet und angeordnet ist, dass eine Rotation der Knochenschraube (3) in einer Eindrehrichtung (E) eine Lateralbewegung der Knochenschraube (3) in Richtung des Verblockungsbereiches (7) bewirkt,
**dadurch gekennzeichnet, dass**
die Kompressionskontur (6) als Eingriffskontur (6) ausgebildet ist, so dass die Lateralbewegung zumindest teilweise, insbesondere vollständig, durch einen Eingriff zwischen der Eingriffskontur (6) und einer Gegenkontur (9) am Schraubenkopf (4) erzeugbar ist.

2. Knochenplatte (1) gemäss Anspruch 1,
**dadurch gekennzeichnet, dass**
die Lateralbewegung zumindest teilweise, insbesondere vollständig, durch ein Abwälzen des Schraubenkopfes (4) entlang der Eingriffskontur (6) erzeugbar ist.

3. Knochenplatte (1) gemäss einem der vorangehenden Ansprüche,
**dadurch gekennzeichnet, dass**
die Eingriffskontur (6) mindestens eine Führungsfläche (10) aufweist, entlang welcher während der Rotation der Knochenschraube (3) in ihrer Eindrehrichtung (E) eine Unterseite (11) des Schraubenkopfes (4), insbesondere eine Unterseite (11) eines radial am Schraubenkopf (4) angeordneten Verblockungsvorsprunges (12), führbar ist, insbesondere entlang einer Längsachse (M) der Knochenschraube (3).

4. Knochenplatte (1) gemäss einem der vorangehenden Ansprüche,
**dadurch gekennzeichnet, dass**
die Eingriffskontur (6) mindestens einen Abwälzbereich (13) aufweist, an dem ein radial am Schraubenkopf (4) angeordneter Verblockungsvorsprung (12') während zumindest eines Teils der Lateralbewegung abwälzbar ist.

5. Knochenplatte (1) gemäss Anspruch 4,
**dadurch gekennzeichnet, dass**
der Abwälzbereich (13) eine Bodenfläche (21) und eine zwischen der Oberseite (26) und der Bodenfläche (21) verlaufende Seitenfläche (22) enthält, wobei der radial am Schraubenkopf (4) angeordnete Verblockungsvorsprung (12') während zumindest eines Teils der Lateralbewegung an dieser Seitfläche (22) abwälzbar ist.

6. Knochenplatte (1) gemäss einem der vorangehenden Ansprüche,
**dadurch gekennzeichnet, dass**
die Verblockungskontur (8) derart ausgebildet ist, dass die Knochenschraube (3) unter verschiedenen Winkeln relativ zur Knochenplatte (1) verblockbar ist.

7. Knochenplatte (1) gemäss einem der vorangehenden Ansprüche,
**dadurch gekennzeichnet, dass**
die Verblockungskontur (8) mindestens eine Ausnehmung (16) aufweist, die sich keilförmig nach aussen von einer Hauptachse (L) des Verblockungsbereiches (7) weg erweitert, um einen radial am Schraubenkopf (4) angeordneten Verblockungsvorsprung (12, 12', 12") aufnehmen und verblocken zu können.

8. Knochenplatte (1) gemäss Anspruch 7,
**dadurch gekennzeichnet, dass**
die Ausnehmung (16) der Verblockungskontur (8) so angeordnet ist, dass sie in einer Richtung senkrecht zur Hauptachse (L) des Verblockungsbereiches (7) umläuft.

9. Knochenplatte (1) gemäss einem der Ansprüche 7 und 8,
**dadurch gekennzeichnet, dass**
die Verblockungskontur (8) mindestens zwei, bevorzugt mindestens drei und besonders bevorzugt genau drei Ausnehmungen (16) aufweist, die sich jeweils keilförmig nach aussen von der Hauptachse (L) des Verblockungsbereiches (7) weg erweitern.

10. Operationsset, enthaltend
- mindestens eine Knochenplatte (1) gemäss einem der vorangehenden Ansprüche
- mindestens eine Knochenschraube (3) mit einem Schraubenkopf (4), welcher eine Gegenkontur umfasst, und welche in mindestens einer der Aufnahmeöffnungen (2) der Knochenplatte (1) einsetzbar ist.

11. Operationsset gemäss Anspruch 10, **dadurch gekennzeichnet, dass**
- die mindestens eine Knochenplatte (1) eine Knochenplatte gemäss einem der Ansprüche 3 bis 9 ist
- die Gegenkontur (9) durch mindestens einen der Verblockungsvorsprünge (12, 12', 12") und/oder mindestens eine der zwischen den Verblockungsvorsprüngen (12, 12', 12") angeordneten Ausnehmungen (15, 15', 15") gebildet ist.

12. Operationsset gemäss einem der Ansprüche 10 oder 11,
**dadurch gekennzeichnet, dass**
der Schraubenkopf (4) mit einem radial am Schraubenkopf (4) angeordneten Verblockungsvorsprung (12, 12', 12") mit einer umlaufenden Aussenfläche (17) versehen ist, die sich im Wesentlichen in Richtung einer Längsachse (M) der Knochenschraube (3) erstreckt und mindestens eine Klemmfläche (18) aufweist, welche sich, in einer Azimutebene (A) senkrecht zur Längsachse (M) betrachtet, keilförmig nach aussen von der Längsachse (M) weg erweitert, um den Schraubenkopf (4) mit der Verblockungskontur (8) verblocken zu können.

13. Operationsset gemäss Anspruch 12,
**dadurch gekennzeichnet, dass**
die umlaufende Aussenfläche (17) des Schraubenkopfes (4) mindestens drei, bevorzugt genau drei gleichmässig entlang ihres Umfangs verteilte Klemmflächen (18) aufweist, die sich jeweils keilförmig nach aussen von der Längsachse (M) der Knochenschraube (3) weg erweitern.

14. Operationsset gemäss einem der Ansprüche 10 bis 13,
**dadurch gekennzeichnet, dass**
mindestens eine Knochenplatte gemäss einem der Ansprüche 3 bis 9 ausgebildet ist und die Führungsfläche (10) derart auf die Knochenschraube (3) abgestimmt ist, insbesondere auf eine Unterseite (11) des Schraubenkopfes (4) und eine Gewindesteigung eines Schaftes (19) der Knochenschraube (3), dass die Unterseite (11) des Schraubenkopfes (4), insbesondere eine Unterseite (11) eines radial am Schraubenkopf (4) angeordneten Verblockungsvorsprunges (12), während des Eindrehens der Knochenschraube (3) entlang der Führungsfläche (10) führbar ist, insbesondere entlang einer Längsachse (M) der Knochenschraube (3).

15. Operationsset gemäss einem der Ansprüche 10 bis 14,
**dadurch gekennzeichnet, dass**
mindestens eine Knochenplatte gemäss einem der Ansprüche 4 bis 9 ausgebildet ist und der Abwälzbereich (13) dieser Knochenplatte, insbesondere dessen Seitenfläche (22), derart auf die Knochenschraube (3) abgestimmt ist, insbesondere auf eine am Schraubenkopf (4) angeordnete Gegenkontur (9) und eine Gewindesteigung eines Schaftes (19) der Knochenschraube (3), dass der Schraubenkopf (4) während des Eindrehens der Knochenschraube (3) am Abwälzbereich (13), insbesondere an dessen Seitenfläche (22), abwälzbar ist.

## Claims

1. A bone plate (1) with at least one receiving opening (2) for a bone screw (3) with a screw head (4), wherein the receiving opening (2) includes a compression region (5) with a compression contour (6) and a blocking region (7) with a blocking contour (8), wherein the compression contour (6) is realized and arranged in such a manner that rotation of the bone screw (3) in a screwing-in direction (E) brings about a lateral movement of the bone screw (3) in the direction of the blocking region (7),
**characterized in that**
the compression contour (6) is realized as an engagement contour (6) such that the lateral movement is generatable at least in part, in particular completely, by engagement between the engagement contour (6) and a counter contour (9) on the screw head (4).

2. The bone plate (1) as claimed in claim 1,
**characterized in that**
the lateral movement is generatable at least in part, in particular completely, by rolling the screw head (4) along the engagement contour (6).

3. The bone plate (1) as claimed in either of the preceding claims,
**characterized in that**
the engagement contour (6) comprises at least one guiding surface (10), along which is guidable, during rotation of the bone screw (3) in its screwing-in direction (E), a bottom surface (11) of the screw head (4), in particular a bottom surface (11) of a blocking projection (12) which is arranged radially on the screw head (4), in particular along a longitudinal axis (M) of the bone screw (3).

4. The bone plate (1) as claimed in one of the preceding claims, **characterized in that**
the engagement contour (6) comprises at least one rolling region (13) on which a blocking projection (12'), which is arranged radially on the screw head (4), is rollable during at least part of the lateral movement.

5. The bone plate (1) as claimed in claim 4,
**characterized in that**
the rolling region (13) includes a bottom surface (21) and a side surface (22), which extends between the top surface (26) and the bottom surface (21), wherein the blocking projection (12'), which is arranged radially on the screw head (4), is rollable on said side surface (22) during at least part of the lateral movement.

6. The bone plate (1) as claimed in one of the preceding claims,
**characterized in that**
the blocking contour (8) is realized in such a manner that the bone screw (3) is blockable at various angles relative to the bone plate (1).

7. The bone plate (1) as claimed in one of the preceding claims,
**characterized in that**
the blocking contour (8) comprises at least one recess (16) which widens outward in a wedge-shaped manner away from a main axis (L) of the blocking region (7) in order to be able to receive and block a blocking projection (12, 12', 12") which is arranged radially on the screw head (4).

8. The bone plate (1) as claimed in claim 7,
**characterized in that**
the recess (16) of the blocking contour (8) is arranged such that it extends around in a direction perpendicular to the main axis (L) of the blocking region (7).

9. The bone plate (1) as claimed in one of claims 7 and 8,
**characterized in that**
the blocking contour (8) comprises at least two, in a preferred manner at least three and in a particularly preferred manner precisely three recesses (16) which widen outward in each case in a wedge-shaped manner away from the main axis (L) of the blocking region (7).

10. A surgical kit, including
- at least one bone plate (1) as claimed in one of the preceding claims
- at least one bone screw (3) with a screw head (4) comprising a counter contour wherein the bone screw (3) is insertable into at least one of the receiving openings (2) of the bone plate (1).

11. The surgical kit as claimed in claim 10, **characterized in that**
- the at least one bone plate (1) is a bone plate as claimed in one of claims 3 to 9
- the counter contour (9) is realized by at least one of the blocking projections (12, 12', 12") and/or at least one of the recesses (15, 15', 15") arranged between the blocking projections (12, 12', 12").

12. The surgical kit as claimed in one of claims 10 or 11, **characterized in that**
the screw head (4) is provided with a blocking projection (12, 12', 12"), which is arranged radially on the screw head (4) and has a circumferential outside surface (17) which extends substantially in the direction of a longitudinal axis (M) of the bone screw (3) and comprises at least one clamping surface (18) which, when viewed in an azimuth plane (A) perpendicular to the longitudinal axis (M), widens outward in a wedge-shaped manner away from the longitudinal axis (M) in order to be able to block the screw head (4) by way of the blocking contour (8).

13. The surgical kit as claimed in claim 12,
**characterized in that**
the circumferential outside surface (17) of the screw head (4) comprises at least three, in a preferred manner precisely three clamping surfaces (18) which are distributed equally along their circumference and widen outward in each case in a wedge-shaped manner away from the longitudinal axis (M) of the bone screw (3).

14. The surgical kit as claimed in one of claims 10 to 13,
**characterized in that**
at least one bone plate is realized according to one of claims 3 to 9 and the guiding surface (10) is matched to the bone screw (3) in such a manner, in particular to a bottom surface (11) of the screw head (4) and to a thread pitch of a shank (19) of the bone screw (3), that the bottom surface (11) of the screw head (4), in particular a bottom surface (11) of a blocking projection (12) which is arranged radially on the screw head (4), is guidable along the guiding surface (10), in particular along a longitudinal axis (M) of the bone screw, during the screwing-in of the bone screw (3) .

15. The surgical kit as claimed in one of claims 10 to 14,
**characterized in that**
at least one bone plate is realized according to one of claims 4 to 9 and the rolling region (13) of said bone plate, in particular of the side surface (22) thereof, is matched in such a manner to the bone screw (3), in particular to a counter contour (9) which is arranged on the screw head (4) and to a thread pitch of a shank (19) of the bone screw (3), that the screw head (4) is rollable on the rolling region (13), in particular on the side surface (22) thereof, during the screwing-in of the bone screw (3).

## Revendications

1. Plaque d'ostéosynthèse (1) avec au moins une ouverture de logement (2) pour une vis d'ostéosynthèse (3) avec une tête de vis (4), l'ouverture de logement (2) contenant une zone de compression (5) avec un profil de compression (6) et une zone de blocage (7) avec un profil de blocage (8), le profil de compression (6) étant constitué et disposé de telle manière qu'une rotation de la vis d'ostéosynthèse (3) dans une direction de vissage (E) cause un mouvement latéral de la vis d'ostéosynthèse (3) en direction de la zone de blocage (7),
**caractérisée en ce que**
le profil de compression (6) est constituée sous la forme d'un profil d'engagement (6) telle sorte que le mouvement latéral peut être produit au moins en partie, en particulier complètement, par un engagement entre le profil d'engagement (6) et un contre-profil (9) sur la tête de vis (4).

2. Plaque d'ostéosynthèse (1) selon la revendication 1, **caractérisée en ce que**
le mouvement latéral peut être produit au moins en partie, en particulier complètement, par un roulement de la tête de vis (4) le long du profil d'engagement (6) .

3. Plaque d'ostéosynthèse (1) selon l'une quelconque des revendications précédentes,
**caractérisée en ce que**
le profil d'engagement (6) comporte au moins une surface de guidage (10), le long de laquelle pendant la rotation de la vis d'ostéosynthèse (3) dans sa direction de vissage (E), une face inférieure (11) de la tête de vis (4), en particulier une face inférieure (11) d'une saillie de blocage (12) disposée radialement sur la tête de vis (4), peut être guidée, en particulier le long d'un axe longitudinal (M) de la vis d'ostéosynthèse (3).

4. Plaque d'ostéosynthèse (1) selon l'une quelconque des revendications précédentes,
**caractérisée en ce que**
le profil d'engagement (6) comporte au moins une zone de roulement (13) sur laquelle une saillie de blocage (12') disposée radialement sur la tête de vis (4) peut être déroulée pendant au moins une partie du mouvement latéral.

5. Plaque d'ostéosynthèse (1) selon la revendication 4,
**caractérisée en ce que**
la zone de roulement (13) contient une surface de fond (21) et une surface latérale (22) passant entre la surface supérieure (26) et la surface de fond (21), la saillie de blocage (12') étant disposée radialement sur la tête de vis (4) peut être roulée sur cette surface latérale (22) pendant au moins une partie du mouvement latéral.

6. Plaque d'ostéosynthèse (1) selon l'une quelconque des revendications précédentes,
**caractérisée en ce que**
le profil de blocage (8) est constitué de telle manière que la vis d'ostéosynthèse (3) peut être bloquée sous différents angles par rapport à la plaque d'ostéosynthèse (1).

7. Plaque d'ostéosynthèse (1) selon l'une quelconque des revendications précédentes, **caractérisée en ce que** le profil de blocage (8) comporte au moins un évidement (16), qui s'élargit de façon cunéiforme vers l'extérieur en s'éloignant de l'axe principal (L) de la zone de blocage (7) pour pouvoir loger et bloquer une saillie de blocage (12, 12', 12") disposée radialement sur la tête de vis (4) .

8. Plaque d'ostéosynthèse (1) selon la revendication 7,
**caractérisée en ce que**
l'évidement (16) du profil de blocage (8) est disposé de telle manière qu'il passe périphériquement dans une direction perpendiculaire à l'axe principal (L) de la zone de blocage (7).

9. Plaque d'ostéosynthèse (1) selon l'une quelconque des revendications 7 et 8,
**caractérisée en ce que**
le profil de blocage (8) comporte au moins deux, de préférence au moins trois et en particulier de préférence exactement trois évidements (16), qui s'élargissent respectivement de forme cunéiforme vers l'extérieur en s'éloignant de l'axe principal (L) de la zone de blocage (7).

10. Kit chirurgical, contenant :
- au moins une plaque d'ostéosynthèse (1) selon l'une quelconque des revendications précédentes,
- au moins une plaque d'ostéosynthèse (3) avec une tête de vis (4), laquelle comprend un contre-profil et laquelle peut être insérée dans au moins une des ouvertures de logement (2) de la plaque d'ostéosynthèse (1) .

11. Kit chirurgical selon la revendication 10, **caractérisé en ce que**
- au moins une plaque d'ostéosynthèse (1) est une plaque d'ostéosynthèse selon l'une quelconque des revendications 3 à 9,
- le contre-profil (9) est formé par au moins une des saillies de blocage (12,12',12") et/ou au moins un des évidements (15, 15', 15") disposés entre les saillies de blocage (12, 12', 12").

12. Kit chirurgical selon l'une quelconque des revendications 10 ou 11,
**caractérisé en ce que**
la tête de vis (4) avec une saillie de blocage (12, 12', 12") disposée radialement sur la tête de vis (4) est dotée d'une surface extérieure périphérique (17), qui s'étend pour l'essentiel en direction d'un axe longitudinal (M) de la vis d'ostéosynthèse (3) et comporte au moins une surface de serrage (18), laquelle s'élargit, vue dans un plan azimutal (A) perpendiculairement à l'axe longitudinal (M), de façon cunéiforme vers l'extérieur en s'éloignant de l'axe longitudinal (M) pour pouvoir bloquer la tête de vis (4) avec le profil de blocage (8).

13. Kit chirurgical selon la revendication 12,
**caractérisé en ce que**
la surface extérieure périphérique (17) de la tête de vis (4) comporte au moins trois, de préférence exactement trois surfaces de serrage (18) réparties uniformément le long de sa périphérie, qui s'élargissent respectivement de façon cunéiforme vers l'extérieur en s'éloignant de l'axe longitudinal (M) de la vis d'ostéosynthèse (3).

14. Kit chirurgical selon l'une quelconque des revendications 10 à 13,
**caractérisé en ce qu'**
au moins une plaque d'ostéosynthèse est constituée selon l'une quelconque des revendications 3 à 9 et la surface de guidage (10) est accordée à la vis d'ostéosynthèse (3), en particulier à une face inférieure (11) de la tête de vis (4) et un pas de filetage d'une tige (19) de la vis d'ostéosynthèse (3) de telle manière que la face inférieure (11) de la tête de vis (4), en particulier une face inférieure (11) d'une saillie de blocage (12) disposée radialement sur la tête de vis (4), alors que le vissage de la vis d'ostéosynthèse (3) peut être guidée le long de la surface de guidage (10), en particulier le long d'un axe longitudinal (M) de la vis d'ostéosynthèse (3).

15. Kit chirurgical selon l'une quelconque des revendications 10 à 14,
**caractérisé en ce qu'**
au moins une plaque d'ostéosynthèse est constituée selon l'une quelconque des revendications 4 à 9 et la zone de roulement (13) de cette plaque d'ostéosynthèse, en particulier de sa surface latérale (22), est accordée à la vis d'ostéosynthèse (3) en particulier à un contre-profil (9) disposé sur une tête de vis (4) et un pas de filetage d'une tige (19) de la vis d'ostéosynthèse (3) de telle manière que la tête de vis (4) peut être roulée sur la zone de roulement (13), en particulier sur sa surface latérale (22), pendant le vissage de la vis d'ostéosynthèse (3).
